# EUROPEAN PATENT SPECIFICATION

(11) **EP 3 992 301 B1**
(45) Date of publication and mention of the grant of the patent: **11.02.2026**
(21) Application number: 19935362.4
(22) Date of filing: 28.06.2019
(51) Int. Cl.: C12Q 1/6813, C12Q 1/32, G01N 33/50, G01N 33/58, G01N 33/543

(54) **METHOD FOR ASSESSING DIFFERENTIATION STATE OF CELLS AND GELATIN NANOPARTICLES**
VERFAHREN ZUR BEURTEILUNG DES DIFFERENZIERUNGSZUSTANDS VON ZELLEN UND GELATINENANOPARTIKELN
PROCÉDÉ D'ÉVALUATION DE L'ÉTAT DE DIFFÉRENCIATION DE CELLULES ET DE NANOPARTICULES DE GÉLATINE

(43) Date of publication of application: 04.05.2022
(73) Proprietor: KONICA MINOLTA, INC., Tokyo 100-7015 (JP)
(72) Inventor: ITO, Temmei, Tokyo 100-7015 (JP); MAEZAWA, Akihiro, Tokyo 100-7015 (JP); TABATA, Yasuhiko, Uji-city, Kyoto 611-0024 (JP); MURATA, Yuki, Kyoto-city Kyoto 606-8507 (JP)
(74) Representative: Gille Hrabal Partnerschaftsgesellschaft mbB Patentanwälte
(86) International application number: PCT/JP2019/025952
(87) International publication number: WO 2020/261569

(56) References cited:
- WO-A1-2009/119647
- WO-A1-2017/110746
- WO-A1-2018/031407
- HALVARSSON CAMILLA ET AL: "Pyruvate dehydrogenase kinase 1 is essential for transplantable mouse bone marrow hematopoietic stem cell and progenitor function", PLOS ONE, vol. 112, no. 2, 9 February 2017 (2017-02-09), pages e0171714, XP055778222, DOI: 10.1371/journal.pone.0171714
- HALVERSSON CAMILLA: "S2 Table", 9 February 2017 (2017-02-09), XP055929018, Retrieved from the Internet <URL:https://www.ncbi.nlm.nih.gov/pmc/articles/PMC5300157/> [retrieved on 20220608]
- HE JUNMING ET AL: "Stage-specific requirement of kinase PDK1 for NK cells development and activation", CELL DEATH & DIFFERENTIATION, NATURE PUBLISHING GROUP, GB, vol. 26, no. 10, 8 January 2019 (2019-01-08), pages 1918 - 1928, XP036878934, ISSN: 1350-9047, [retrieved on 20190108], DOI: 10.1038/S41418-018-0263-8
- PENG F ET AL: "Glycolysis gatekeeper PDK1 reprograms breast cancer stem cells under hypoxia", vol. 37, no. 8, 22 February 2018 (2018-02-22), London, pages 1062 - 1074, XP055928985, ISSN: 0950-9232, Retrieved from the Internet <URL:https://www.ncbi.nlm.nih.gov/pmc/articles/PMC5851116/pdf/onc2017368a.pdf> DOI: 10.1038/onc.2017.368
- MURATA YUKI ET AL: "Preparation of cationized gelatin nanospheres incorporating molecular beacon to visualize cell apoptosis", vol. 8, no. 1, 4 October 2018 (2018-10-04), XP055778242, Retrieved from the Internet <URL:http://www.nature.com/articles/s41598-018-33231-2> DOI: 10.1038/s41598-018-33231-2
- HALVARSSON, C. ET AL.: "Pyruvate dehydrogenase kinase 1 is essential for transplantable mouse bone marrow hematopoietic stem cell and progenitor function", PLOS ONE, vol. 12, no. 2, e0171714, 2017, pages 1 - 17, XP055778222
- RODRIGUES, A. S. ET AL.: "Dichloroacetate, the Pyruvate Dehydrogenase Complex and the Modulation of mESC Pluripotency", PLOS ONE, vol. 10, no. 7, e0131663, 2015, pages 1 - 18, XP055778223
- TAKUBO, K. ET AL.: "Regulation of Glycolysis by Pdk Functions as a Metabolic Checkpoint for Cell Cycle Quiescence in Hematopoietic Stem Cells", CELL STEM CELL, vol. 12, 2013, pages 49 - 61, XP055778232
- SHINKYO YOON, JONG GWANG KIM, A. SEO, S. PARK, HYE JIN KIM, JUN SEOK PARK, G. CHOI, J. JEONG, D. JUN, G. YOON, B. KANG: "Clinical Implication of Serine Metabolism-Associated Enzymes in Colon Cancer", ONCOLOGY, vol. 89, no. 6, 26 November 2015 (2015-11-26), pages 351 - 359, XP009533294, ISSN: 0030-2414, DOI: 10.1159/000439571
- GUGLIELMETTI, C. ET AL.: "Hyperpolarized 13C MR metabolic imaging can detect neuroinflammation in vivo in a multiple sclerosis murine model", PNAS, 2017, pages E6982 - E6991, XP055778238
- MURATA, Y. ET AL.: "Preparation of cationized gelatin nanospheres incorporating molecular beacon to visualize cell apoptosis", SCIENTIFIC REPORTS, vol. 8, no. 14839, 2018, pages 1 - 11, XP055778242
- FUNATSU TAKASHI: "Analysis of cellular functions by specific fluorescence labeling and imaging of mRNAs in living cells", DRUG DELIVERY SYSTEM, vol. 31, no. 2, 25 March 2016 (2016-03-25), pages 110 - 118, XP055885749, ISSN: 0913-5006, DOI: 10.2745/dds.31.110

## Description

### Technical Field

The present invention relates to a method for assessing the differentiation state of a cell and a gelatin nanoparticle.

### Background Art

In various fields such as regenerative medicine and disease detection and treatment, there is a demand for understanding the differentiation state of cells.

Conventionally, the assessing of the differentiation state of cells has been performed by immunostaining or quantification of the expression level of a marker gene. However, these methods cannot assess the differentiation state of cells over time, nor can they assess the differentiation state of individual cells.

Patent Literature (hereinafter, referred to as PTL) 1 discloses a method for monitoring differentiation of cardiac muscle cells over time by introducing into the cardiac muscle cells a reporter gene for a photoprotein configured to emit light in response to the expression of a cardiac differentiation marker gene. In PTL 1, a vector incorporating the promoter of the marker gene and the gene of a photoprotein (for example, luciferase) located downstream of the promoter is introduced into cells by electroporation. When a transcription factor is synthesized and the above cardiac differentiation marker gene is expressed, the photoprotein derived from the above vector is also expressed and emits light. PTL 1 teaches that observing the emitted light can monitor the differentiation of the cardiac muscle cells.

### Citation List

### Patent Literature

PTL 1
Japanese Patent Application Laid-Open No. 2015-77122

### Summary of Invention

### Technical Problem

There is a demand, as PTL 1 also recognizes, for developing a method capable of assessing cell differentiation over time.

A method that uses a differentiation marker gene specific to a particular cell as described in PTL 1 can assess only the differentiation state of the particular cell. Therefore, for assessing the differentiation state of other cells, it is necessary to search for differentiation marker genes that can be used to assess the differentiation state of such other cells.

The present invention has been made based on the above findings. An object of the present invention is to provide a method for assessing the differentiation state of cells-the method capable of assessing the differentiation state of a wide variety of cells-and gelatin nanoparticles that can be used in the method.

### Solution to the Problem:

The solution to the above object is achieved by the invention as defined in the appended claims.

### Advantageous Effects of Invention

The present invention provides a method for assessing the differentiation state of cells-the method capable of assessing the differentiation state of a wide variety of cells-and gelatin nanoparticles that can be used in the method.

### Brief Description of Drawing

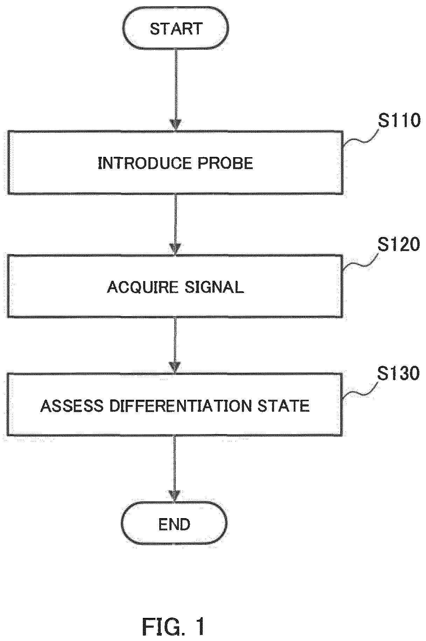

FIG. 1 is a flowchart showing a method for assessing the differentiation state of cells according to an embodiment of the present invention;

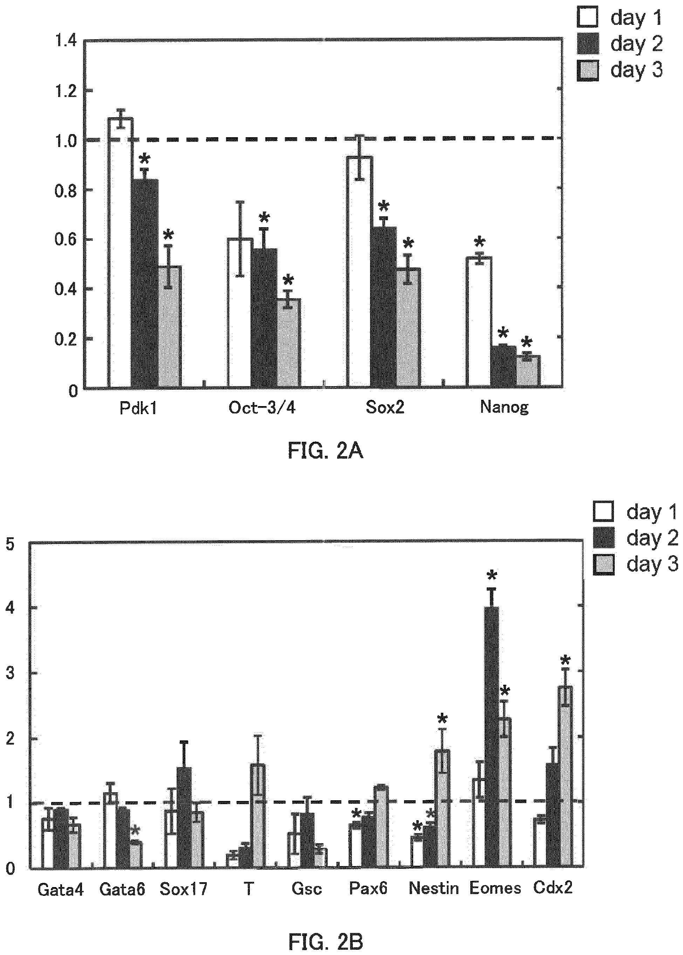

FIG. 2A is a graph showing the expression levels of the mRNA of Pdk1 and undifferentiation markers in Test 1, and FIG. 2B is a graph showing the expression levels of mRNA of the early differentiation markers in Test 1;

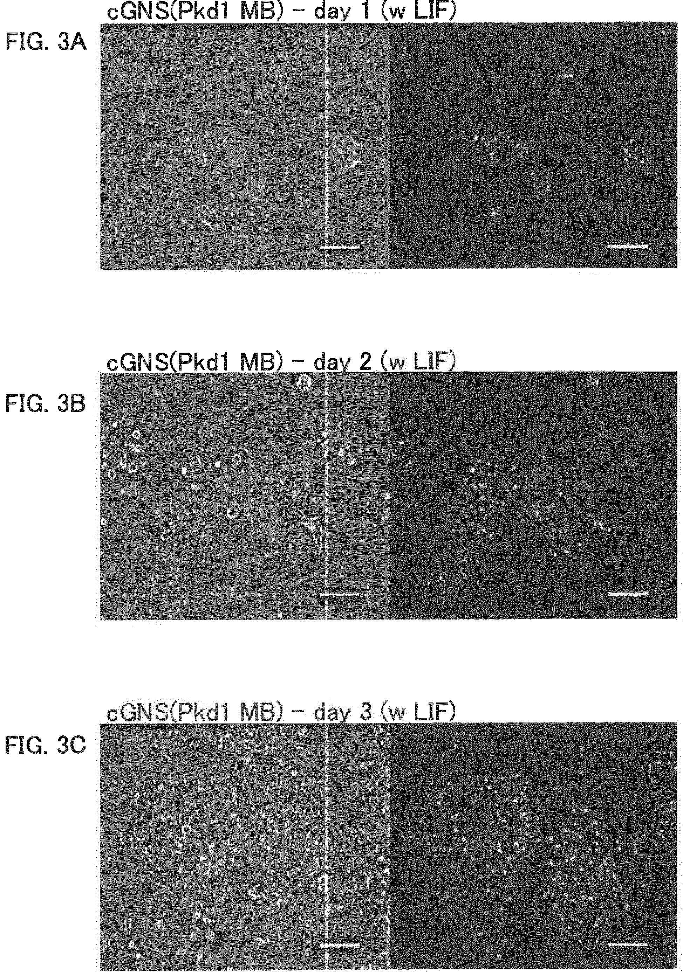

FIG. 3A is a fluorescence image (right side) of a medium one day after the addition of cGNS (Pdk1 MB) under the condition with LIF addition in Test 1 and an image (left side) in which a bright field image and the fluorescence image are superimposed, FIG. 3B is a fluorescence image (right side) of a medium two days after the addition of cGNS (Pdk1 MB) under the condition with LIF addition in Test 1 and an image (left side) in which a bright field image and the fluorescence image are superimposed, and FIG. 3C is a fluorescence image (right side) of a medium three days after the addition of cGNS (Pdk1 MB) under the condition with LIF addition in Test 1 and an image (left side) in which a bright field image and the fluorescence image are superimposed;

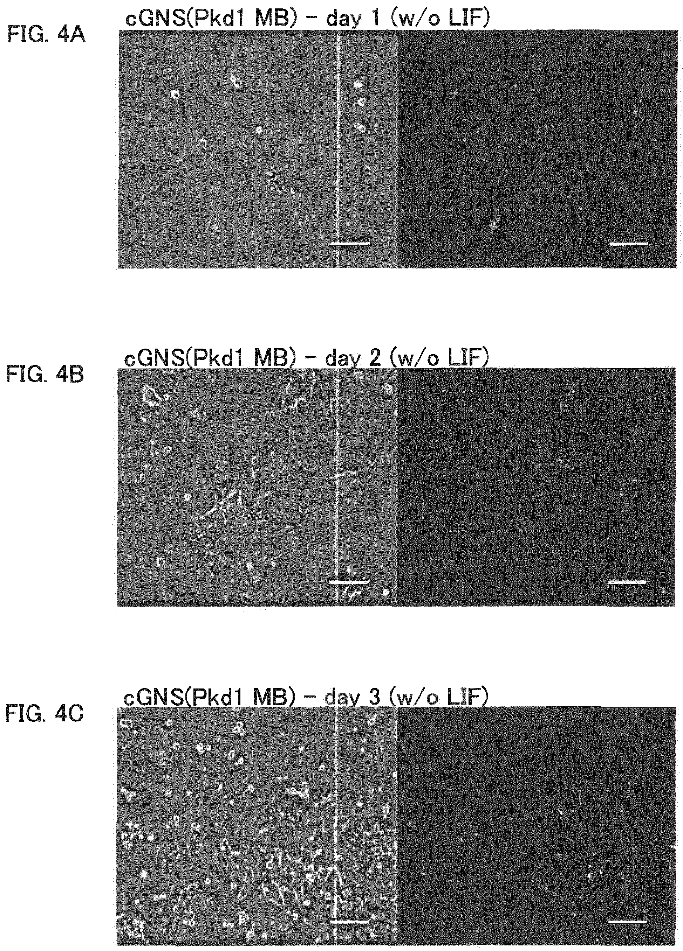

FIG. 4A is a fluorescence image (right side) of a medium one day after the addition of cGNS (Pdk1 MB) under the condition without LIF addition in Test 1 and an image (left side) in which a bright field image and the fluorescence image are superimposed, FIG. 4B is a fluorescence image (right side) of a medium two days after the addition of cGNS (Pdk1 MB) under the condition without LIF addition in Test 1 and an image (left side) in which a bright field image and the fluorescence image are superimposed, and FIG. 4C is a fluorescence image (right side) of a medium three days after the addition of cGNS (Pdk1 MB) under the condition without LIF addition in Test 1 and an image (left side) in which a bright field image and the fluorescence image are superimposed;

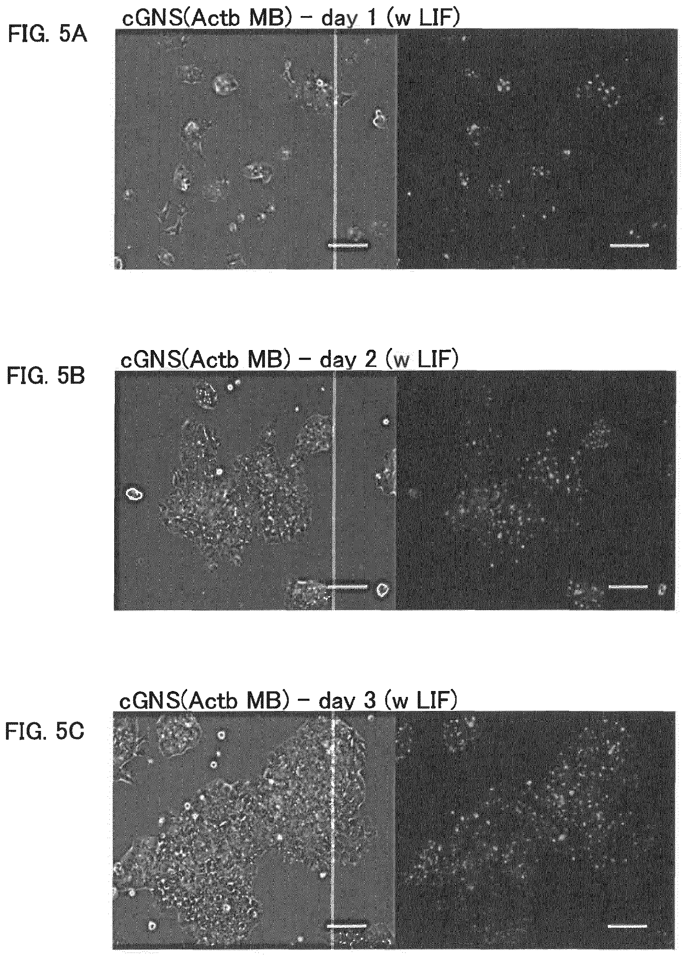

FIG. 5A is a fluorescence image (right side) of a medium one day after the addition of cGNS (Actb MB) under the condition with LIF addition in Test 1 and an image (left side) in which a bright field image and the fluorescence image are superimposed, FIG. 5B is a fluorescence image (right side) of a medium two days after the addition of cGNS (Actb MB) under the condition with LIF addition in Test 1 and an image (left side) in which a bright field image and the fluorescence image are superimposed, and FIG. 5C is a fluorescence image (right side) of a medium three days after the addition of cGNS (Actb MB) under the condition with LIF addition in Test 1 and an image (left side) in which a bright field image and the fluorescence image are superimposed;

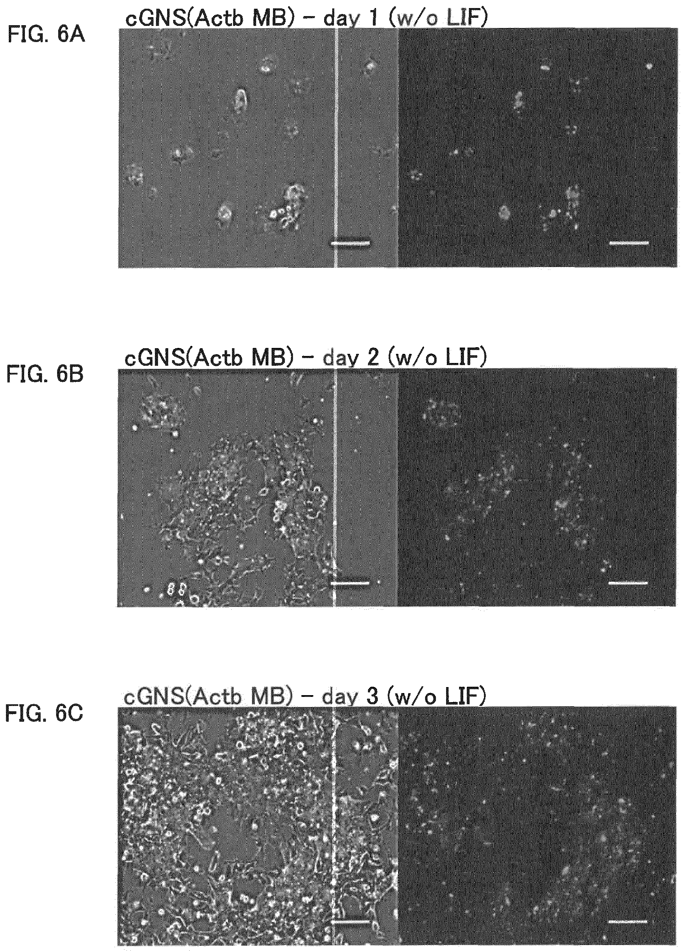

FIG. 6A is a fluorescence image (right side) of a medium one day after the addition of cGNS (Actb MB) under the condition without LIF addition in Test 1 and an image (left side) in which a bright field image and the fluorescence image are superimposed;
FIG. 6B is a fluorescence image (right side) of a medium two days after the addition of cGNS (Actb MB) under the condition without LIF addition in Test 1 and an image (left side) in which a bright field image and the fluorescence image are superimposed, and FIG. 6C is a fluorescence image (right side) of a medium three days after the addition of cGNS (Actb MB) under the condition without LIF addition in Test 1 and an image (left side) in which a bright field image and the fluorescence image are superimposed;

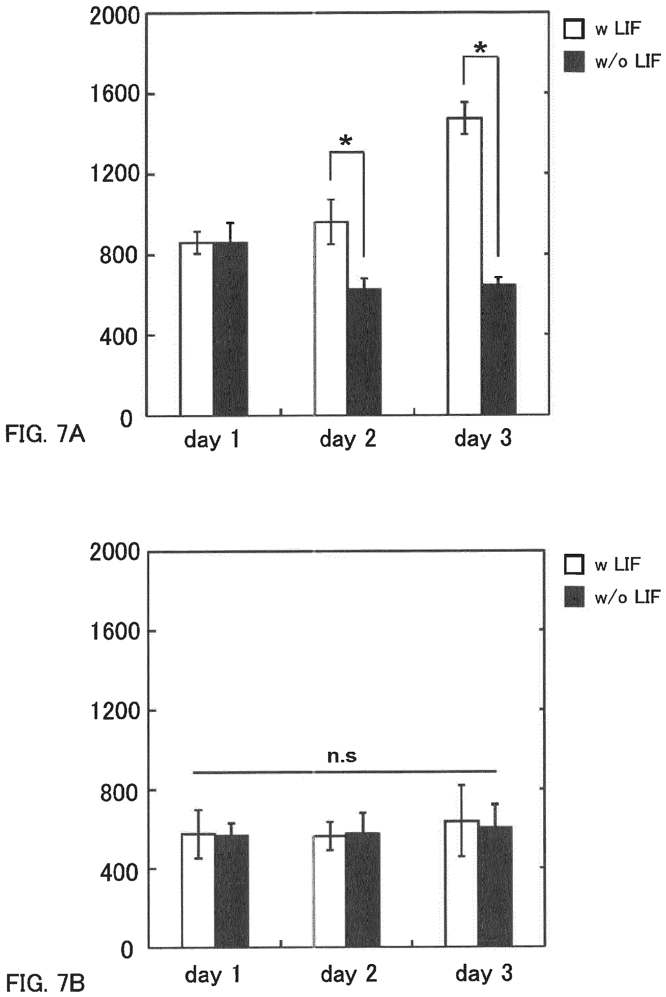

FIG. 7A is a graph showing the fluorescence intensities of mediums with cGNS (Pdk1 MB) added thereto in Test 1, and FIG. 7B is a graph showing the fluorescence intensities of mediums with cGNS (Actb MB) added thereto in Test 1;

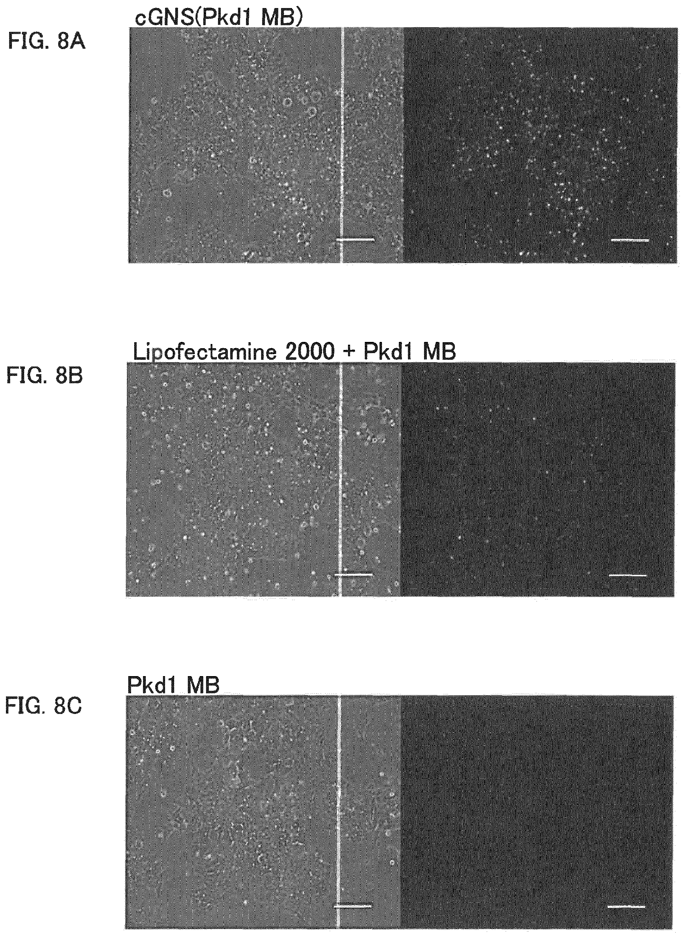

FIG. 8A is a fluorescence image (right side) of a medium with cGNS (Pdk1 MB) added thereto in Test 1 and an image (left side) in which a bright field image and the fluorescence image are superimposed, FIG. 8B is a fluorescence image (right side) of a medium with the complex of Lipofectamine 2000 and Pdk1 MB added thereto in Test 1 and an image (left side) in which a bright field image and the fluorescence image are superimposed, and FIG. 8C is a fluorescence image (right side) of a medium with Pdk1 MB alone added thereto in Test 1 and an image (left side) in which a bright field image and the fluorescence image are superimposed;

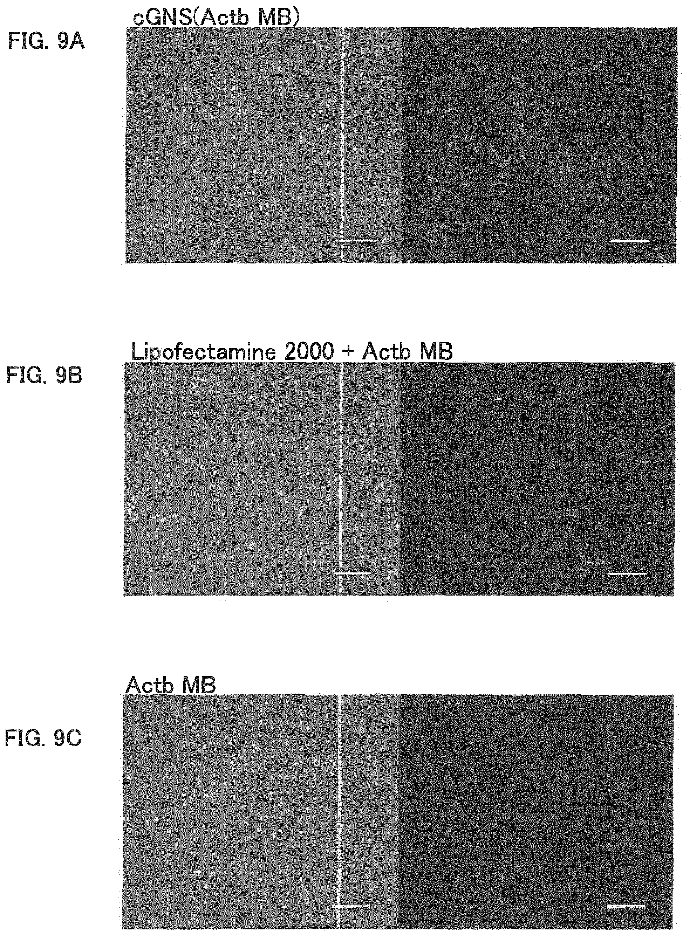

FIG. 9A is a fluorescence image (right side) of a medium with cGNS (Actb MB) added thereto in Test 1 and an image (left side) in which a bright field image and the fluorescence image are superimposed, and FIG. 9B is a fluorescence image (right side) of a medium with the complex of Lipofectamine 2000 and Actb MB added thereto in Test 1 and an image (left side) in which a bright field image and the fluorescence image are superimposed, and FIG. 9C is a fluorescence image (right side) of a medium with Actb MB alone added thereto in Test 1 and an image (left side) in which a bright field image and the fluorescence image are superimposed;

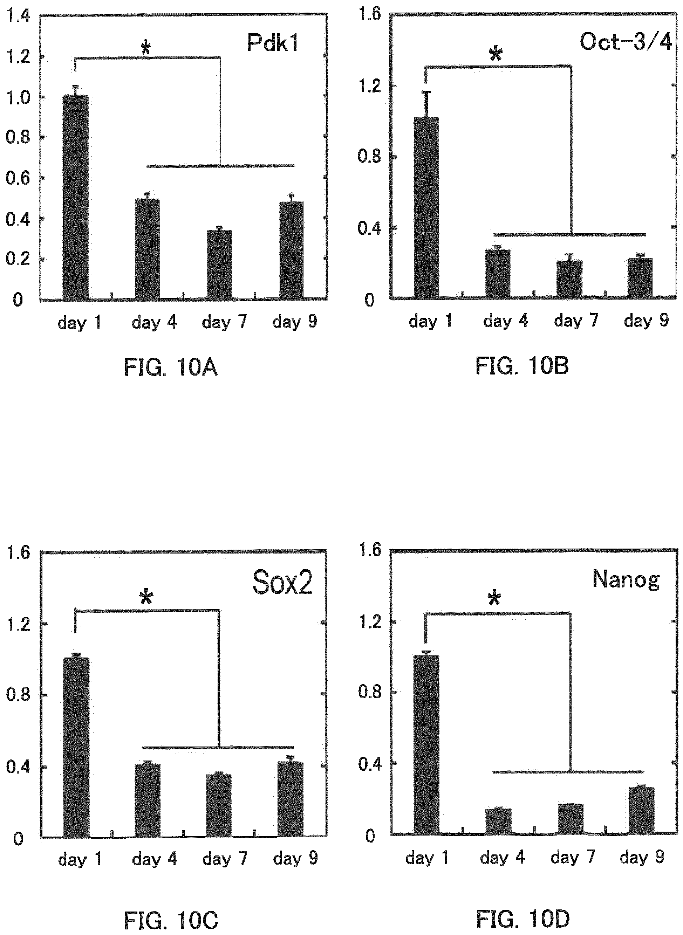

FIG. 10A is a graph showing the expression levels of the mRNA of Pdk1 in Test 2, FIG. 10B is a graph showing the expression levels of the mRNA of Oct-3/4 in Test 2, FIG. 10C is a graph showing the expression levels of the mRNA of Sox2 in Test 2, and FIG. 10D is a graph showing the expression levels of the mRNA of Nanog in Test 2;

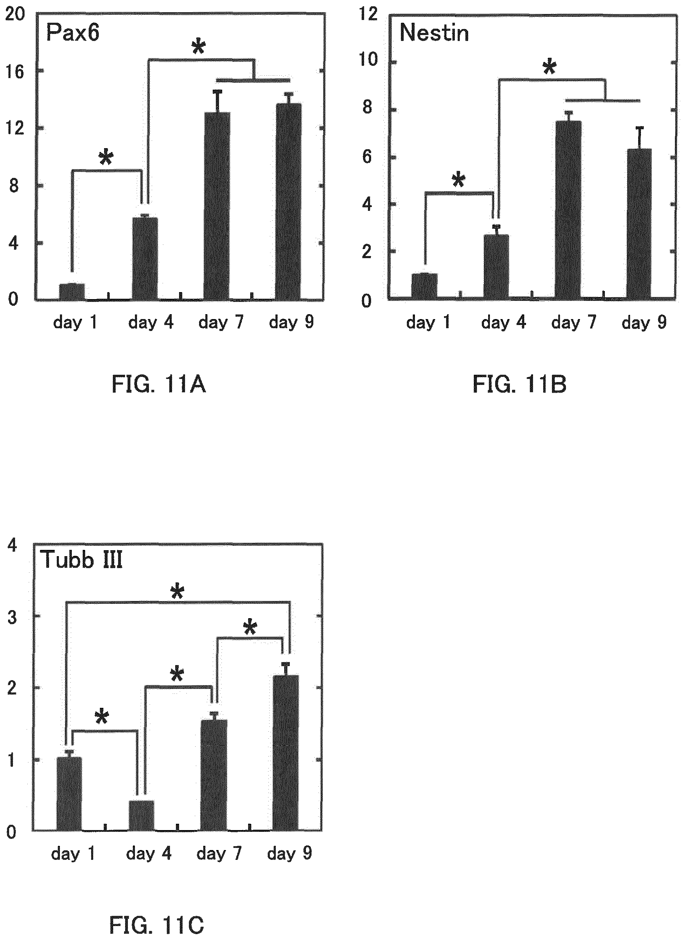

FIG. 11A is a graph showing the expression levels of the mRNA of Pax6 in Test 2, FIG. 11B is a graph showing the expression levels of the mRNA of Nestin in Test 2, and FIG. 11C is a graph showing the expression levels of the mRNA of Tubb III in Test 2;

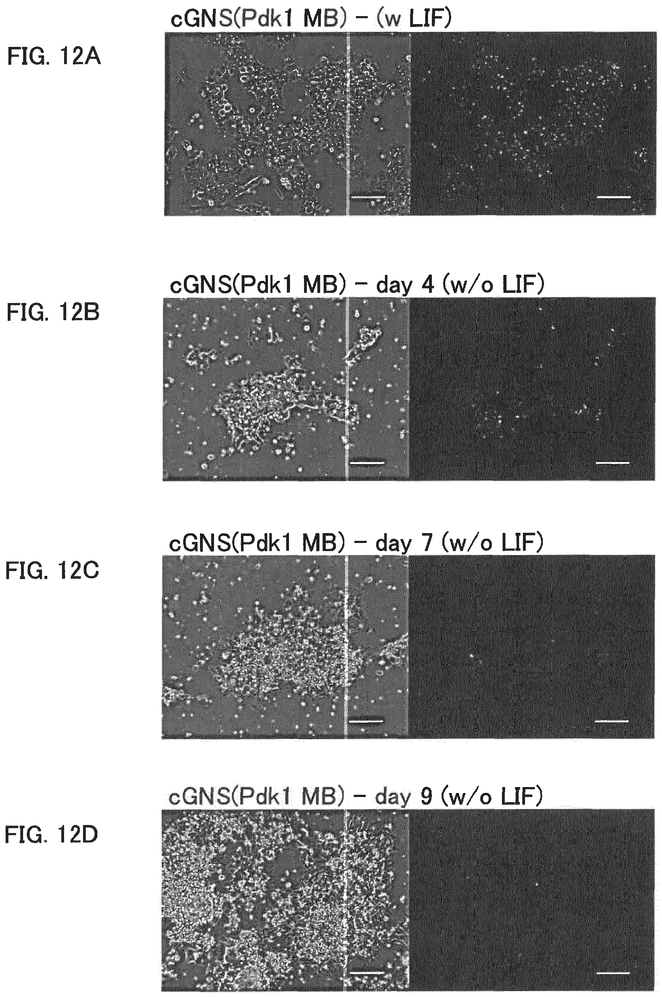

FIG. 12A is a fluorescence image (right side) of a medium with cGNS (Pdk1 MB) added thereto under the condition with LIF addition in Test 2 and an image (left side) in which a bright field image and the fluorescence image are superimposed, FIG. 12B is a fluorescence image (right side) of a medium four days after the addition of cGNS (Pdk1 MB) under the condition without LIF addition in Test 2 and an image (left side) in which a bright field image and the fluorescence image are superimposed, FIG. 12C is a fluorescence image (right side) of a medium seven days after the addition of cGNS (Pdk1 MB) under the condition without LIF addition in Test 2 and an image (left side) in which a bright field image and the fluorescence image are superimposed, and FIG. 12D is a fluorescence image (right side) of a medium nine days after the addition of cGNS (Pdk1 MB) under the condition without LIF addition in Test 2 and an image (left side) in which a bright field image and the fluorescence image are superimposed;

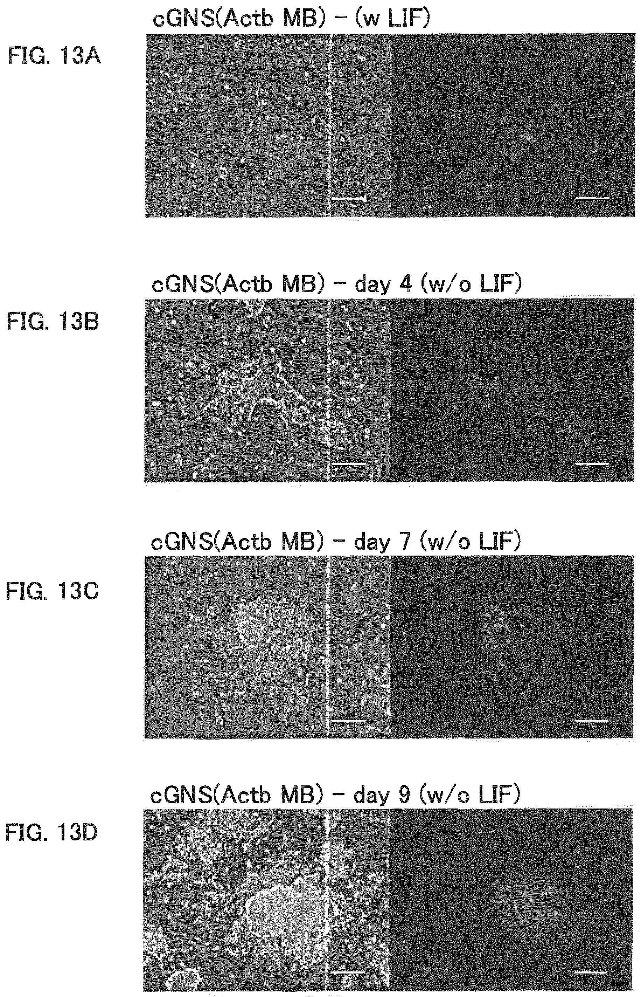

FIG. 13A is a fluorescence image (right side) of a medium with cGNS (Actb MB) added thereto under the condition with LIF addition in Test 2 and an image (left side) in which a bright field image and the fluorescence image are superimposed, FIG. 13B is a fluorescence image (right side) of a medium four days after the addition of cGNS (Actb MB) under the condition without LIF addition in Test 2 and an image (left side) in which a bright field image and the fluorescence image are superimposed, FIG. 13C is a fluorescence image (right side) of a medium seven days after the addition of cGNS (Actb MB) under the condition without LIF addition in Test 2 and an image (left side) in which a bright field image and the fluorescence image are superimposed, and FIG. 13D is a fluorescence image (right side) of a medium nine days after the addition of cGNS (Actb MB) under the condition without LIF addition in Test 2 and an image (left side) in which a bright field image and the fluorescence image are superimposed; and

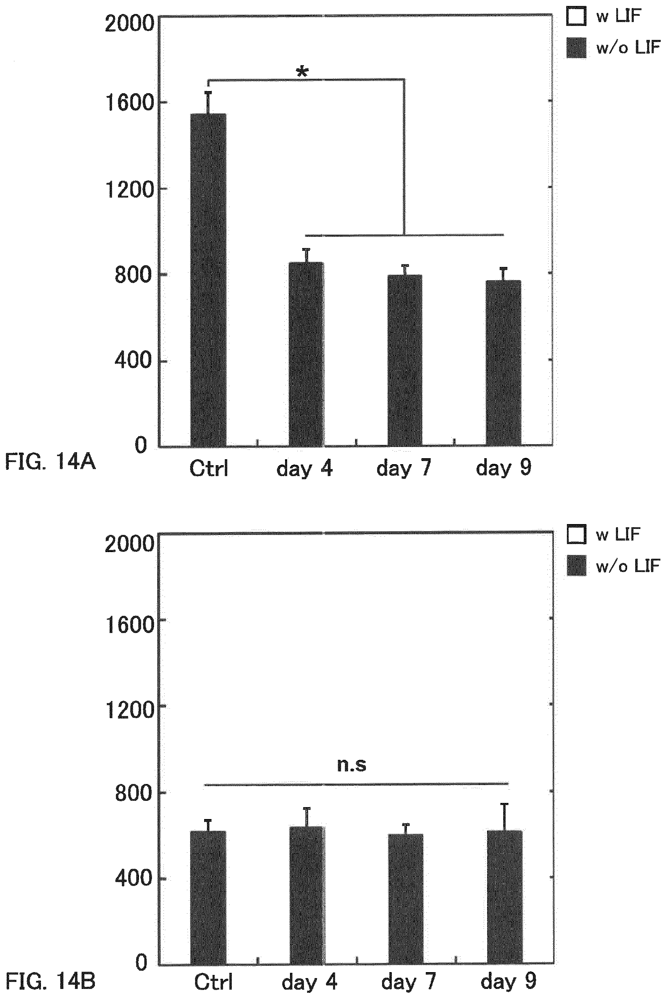

FIG. 14A is a graph showing the fluorescence intensities of mediums with cGNS (Pdk1 MB) added thereto in Test 2, and FIG. 14B is a graph showing the fluorescence intensities of mediums with cGNS (Actb MB) added thereto in Test 2.

### Description of Embodiments

Hereinafter, at least one embodiment of the present invention will be described in detail with reference to the drawings. The present invention is not limited to the following embodiments.

FIG. 1 is a flowchart showing a method for assessing the differentiation state of cells according to an embodiment of the present invention.

In the present embodiment, a probe is introduced into a cell (step S110), a signal from the probe is acquired (step S120), and the differentiation state of the cell is assessed based on the acquired signal (step S130).

### Introduction of Probe (step S110)

First, probes are introduced into cells.

The probe is a probe capable of detecting pyruvate dehydrogenase kinase 1 (PDK1) or mRNA encoding pyruvate dehydrogenase kinase 1 (Pdk1). In the present embodiment, detecting the expression of the enzyme or mRNA detects the metabolic state of cells, thereby assessing the differentiation state of the cells.

Differentiation causes various changes in cells such as size, membrane potential, signal transduction, gene expression, and metabolism. Methods for detecting these changes to assess the differentiation state of cells are studied, but disadvantageously, many of these changes are specific to the cell types after the differentiation, and detection methods need to be studied for each cell type.

The present inventors have decided to focus on the fact that metabolic changes are common to all cell types. The present inventors have further studied based on the idea that detecting this common metabolic change to assess the differentiation state of cells can lead to the development of a method for assessing the differentiation state for a wide variety of cell types.

Cellular metabolism includes glycolysis in cytoplasm, and the TCA cycle and oxidative phosphorylation in mitochondria. It is known that metabolism by glycolysis is predominant in undifferentiated cells, but metabolism in mitochondria (TCA cycle and oxidative phosphorylation) are also activated in somatic cells after differentiation.

The present invention is made from the idea of the present inventors that detecting these metabolic changes can determine the differentiation state of cells. The present inventors have considered that the expression frequency of enzymes involved in the transfer of substances from glycolysis to the TCA cycle changes when cells are differentiated from their undifferentiated state. Then, the present inventors have further studied a method for assessing the differentiation state of cells-from the undifferentiated state in which metabolism by glycolysis is predominant to the post-differentiated state in which metabolism in mitochondria is activated-by the expression of these enzymes.

The final product of glycolysis, namely pyruvate, is oxidatively decarboxylated by a complex composed of pyruvate dehydrogenase (PDH), dihydrolipoamide transacetylase, and dihydrolipoamide dehydrogenase (pyruvate dehydrogenase complex (PDC)), converted to acetyl CoA, and sent to the TCA cycle. PDH is phosphorylated and inhibited in activity by four PDH kinases, namely PDK1, PDK2, PDK3, and PDK4, and dephosphorylated and given activity by two PDH phosphatases, namely pyruvate dehydrogenase phosphatase (PDP) 1 and PDP2.

The above many enzymes and coenzymes thereof are involved in the conversion of pyruvate to acetyl-CoA. However, it was unclear whether the expression of these enzymes or coenzymes would be useful as markers of cell differentiation, and even if these may be used as markers, it was also unclear which enzymes or coenzymes would serve as markers.

The present inventors have found that among the expression of these enzymes, the expression level of PDK1 changes in accordance with cell differentiation. The present inventors have then found that detection of PDK1 expression level is extremely useful for determining the differentiation state of cells from an undifferentiated state in which metabolism by glycolysis is predominant to a post-differentiated state in which metabolism in mitochondria is activated, thereby completing the present invention.

On the basis of the above novel findings, probes capable of detecting Pdk1 or probes capable of detecting PDK1 are introduced into cells to detect the expression of PDK1 in this step.

The probe may be any compound having a site that directly or indirectly binds to Pdk1 or PDK and a site that emits a detectable signal. For example, the probe may be a probe capable of specifically binding to Pdk1 by a nucleic acid having a sequence complementary to at least a part of the nucleic acid sequence of Pdk1, or a probe capable of specifically binding to PDK1 by an antibody. The probe may be a probe that contains a phosphor and emits fluorescence as a signal, or a probe that emits another signal by chemiluminescence or the like.

The phosphor may be of any type, and may be a fluorescent dye or semiconductor nanoparticles.

Examples of the fluorescent dye include rhodamine dye molecules, squarylium dye molecules, fluorescein dye molecules, coumarin dye molecules, acridine dye molecules, pyrene dye molecules, erythrosine dye molecules, eosine dye molecules, cyanine dye molecules, aromatic ring dye molecules, oxazine dye molecules, carbopyronine dye molecules, and pyromethene dye molecules.

Examples of semiconductors constituting the semiconductor nanoparticles include group II-VI compound semiconductors, group III-V compound semiconductors, and group IV semiconductors. Specific examples of the semiconductors constituting semiconductor nanoparticles include CdSe, CdS, CdTe, ZnSe, ZnS, ZnTe, InP, InN, InAs, InGaP, GaP, GaAs, Si, and Ge.

The probe capable of specifically binding to Pdk1 may be a known probe such as a molecular beacon, Taqman probe, cycling probe, or INAF probe, and a molecular beacon is preferred because a general-purpose fluorescent dye can be used, and the detection for various cell types is easy.

A molecular beacon is a nucleic acid derivative having a stem-loop structure, in which a fluorescent dye is bound to one end of the 5' end and the 3' end, and a quenching dye is bound to the other end. When the molecular beacon forms the stem-loop structure described above, the fluorescence emitted from the fluorescent dye is quenched due to the proximity of the fluorescent dye and the quenching dye, but when the molecular beacon comes into close proximity to the target sequence (Pdk1), the loop structure opens and binds to Pdk1. As a result, the fluorescent dye and the quenching dye are separated from each other, and thus fluorescent light emission is detected.

The combination of the fluorescent and the quenching dye is not limited, and the fluorescent can be selected from the fluorescent dyes described above. The quenching dye may be any molecule that quenches by any of fluorescence resonance energy transfer (FRET), contact quenching, and collisional quenching.

The molecular beacon may be in any structure that has a sequence complementary to at least a part of the nucleic acid sequence of Pdk1. The complementary sequence may be any sequence, which is sufficiently complementary such that the molecular beacon can bind to Pdk1, for example, having identity of 80% or more with at least a part of the nucleic acid sequence of Pdk1, preferably identity of 90% or more, more preferably identity of 95% or more. The sequence complementary to at least a part of the nucleic acid sequence of Pdk1 typically forms the loop structure of the above molecular beacon, and may be, for example, a sequence consisting of 2 or more and 40 or less nucleic acids.

The molecular beacon has sequences, which are complementary to each other, respectively on the 5' end side and the 3' end side of the sequence complementary to at least a part of the nucleic acid sequence of Pdk1. These sequences complementary to each other form a stem region of the stem-loop structure by binding to each other. The sequences complementary to each other may be, for example, a sequence consisting of 5 or more and 10 or less nucleic acids. From the viewpoint of enhancing the stability of the molecular beacon, the sequences complementary to each other preferably has the total amount of 50% or more of cytosine (C) and thymine (T) based on the total amount of adenine (A), cytosine (C), thymine (T), and guanine (G).

The probe capable of specifically binding to PDK1 by an antibody is preferably phosphor integrated dots (PID). PIDs are nano-sized particles having a matrix of particles made of organic or inorganic material, and containing a plurality of phosphors. The PID binds directly or indirectly to an antibody that specifically binds to PDK1 to label PDK1. The plurality of phosphors may reside in the particles or on the surface of the particles. The phosphor integrated dots can emit fluorescence of sufficient intensity to indicate the target substance as a bright spot per molecule.

Examples of organic materials for the matrix include thermosetting resins such as melamine resins, urea resins, aniline resins, guanamine resins, phenol resins, xylene resins, and furan resins; thermoplastic resins such as styrene resins, acrylic resins, acrylonitrile resins, AS resins (acrylonitrile-styrene copolymers), and ASA resins (acrylonitrile-styrene-methyl acrylate copolymers); other resins such as polylactic acid; and polysaccharides. Examples of inorganic materials of the matrix include silica and glass. It is preferable that the matrix and the fluorescent substance respectively have substituents or sites having opposite charges, thereby electrostatically interacting each other.

The average particle size of the phosphor integrated dots is not limited, but is preferably 10 nm or more and 500 nm or less, and more preferably 50 nm or more and 200 nm or less from the viewpoint of easy detection as a bright spot.

The particle size of the phosphor integrated dot can be measured by measuring the projected area of the phosphor integrated dot by using a scanning electron microscope (SEM) and converting the area into a circle-equivalent diameter. The average particle size and coefficient of variation of a group of a plurality of phosphor integrated dots are calculated from the particle diameters (circle-equivalent diameters) calculated for a sufficient number (for example, 1,000) of the phosphor integrated dots.

Any method may be used for introducing probes into cells, but it is preferable to introduce the probes into the cells by using gelatin nanoparticles supporting the probes in the present embodiment.

Gelatin nanoparticles are incorporated into the cells by the cells' own activities. Therefore, the gelatin nanoparticles enable the introduction of the above probes into cells in a simplified manner, with less impact on the activity of living cells, compared to other methods such as an electroporation method. In addition, gelatin particles can support a large number of probes, thus can introduce a large number of probes into the cell at one time. Furthermore, the gelatin nanoparticles release the probes slowly over a long period of time after being incorporated into cells, thus enabling the detection of PDK1 or Pdk1 expression over time.

The probe capable of specifically binding to Pdk1 is composed of negatively charged nucleic acids, thus is less likely to enter the negatively charged cell membrane as the probe itself. By supporting the probe in/on gelatin nanoparticle and incorporating the gelatin nanoparticle into the cell, the probe can be introduced into the cell more easily.

The gelatin nanoparticles may be made of any known gelatin that is obtained by denaturing collagen derived from cattle bone, cattle skin, pig skin, pig tendon, fish scales, and fish meat. Gelatin has been conventionally used for foods and for medical purposes, and its intake into the body is hardly harmful to the human body. Further, as gelatin is dispersed and disappears in the living body, gelatin advantageously does not need to be removed from the living body.

The weight average molecular weight of gelatin constituting the gelatin nanoparticles is preferably 1,000 or more and 100,000 or less. The weight average molecular weight may be, for example, a value measured according to the PAGI Method Ver. 10 (2006).

The gelatin constituting the gelatin nanoparticles may be cross-linked. Such cross-linking may be made by a cross-linking agent, or may be self-cross-linking made without any cross-linking agent.

From the viewpoint of facilitating the support of the probes capable of detecting Pdk1, the gelatin nanoparticle is preferably cationized by, for example, introducing a primary amino group, a secondary amino group, a tertiary amino group or a quaternary ammonium group. Nucleic acids have a negative charge thus can electrostatically interact with the cationized gelatin to be bound more strongly.

Gelatin nanoparticles can be cationized by a known method of introducing a functional group that cationizes under physiological conditions during their production. For example, an alkyl diamines such as ethylenediamine or N,N-dimethyl-1,3-diaminopropane, trimethylammonium acetohydrazide, spermine, spermidine, or diethylamide chloride, may be reacted by using a condensing agent such as 1-ethyl-3-(3-dimethylaminopropyl) carbodiimide hydrochloride, cyanuric chloride, N,N' -carbodiimidazole, cyanide bromide, a diepoxy compound, tosyl chloride, a dianhydride compound such as diethyltriamine-N,N,N',N",N"-pentanoate dianhydride, or trityl chloride to introduce the above amino group to the hydroxyl or carboxyl group of gelatin.

The gelatin nanoparticles support the probes. For example, when the probes are molecular beacons, the gelatin nanoparticles support the molecular beacons. When the probes are PIDs, the gelatin nanoparticles support the PIDs, antibodies that specifically bind to PDK1, and medium molecules that bind the antibodies to the PIDs.

Gelatin nanoparticles supporting probes means that the probes are immobilized on the surface of the gelatin nanoparticles or are incorporated into the gelatin nanoparticles.

Gelatin nanoparticles preferably have a larger amount of probes in the inside thereof than the amount of probes in/on the surface layer thereof. By reducing the amount of probes on the surface layer of gelatin nanoparticles, the amount of probes exposed on the surface of gelatin nanoparticles can be reduced. As a result, cells are less likely to recognize the gelatin nanoparticles as foreign substances, and more likely to incorporate the gelatin nanoparticles therein by activities such as endocytosis. The surface layer means a region up to a depth of 1% with respect to the average particle size of gelatin nanoparticles.

The average particle size of the gelatin nanoparticles is preferably 100 nm or more and 1,000 nm or less. Although the gelatin nanoparticles support the probes, the gelatin nanoparticles do not substantially have the probes on the surface layer thereof, so that even with the average particle size of 1,000 nm, the gelatin nanoparticles are more likely to be incorporated into the cells by the cells' own activities. For incorporating many gelatin nanoparticles into cells in a short time, the average particle size of the gelatin nanoparticles is more preferably 800 nm or less. Gelatin nanoparticles whose average particle size is 100 nm or more are more likely to support the probes in the inside of the particles, thereby increasing the capacity for the probes. From the above viewpoint, the average particle size of the gelatin nanoparticles is preferably 200 nm or more, and more preferably 300 nm or more.

The average particle size of the above gelatin nanoparticles can be defined as the apparent particle size of the gelatin nanoparticles, measured by a dynamic light scattering method. Alternatively, the average particle size of the gelatin nanoparticles can be a value obtained by summing and averaging the major axis and the minor axis. The minor axis and major axis of the gelatin nanoparticle(s) can be defined as values obtained by analyzing an image-captured by a scanning electron microscope (SEM)-of the dried gelatin nanoparticle(s) after being allowed to stand in the air at 80°C for 24 hours. Gelatin nanoparticles are usually an aggregate composed of a plurality of gelatin nanoparticles, thus the major axis, the minor axis, and the particle size of the gelatin nanoparticles can be defined as the respective values obtained by summing and averaging the major axes, minor axes, and particle sizes of some gelatin nanoparticles (for example, 20 gelatin nanoparticles) selected from the above aggregate.

The amount of the probes supported by the gelatin nanoparticle(s), the average concentration of the probes on the surface layer of the gelatin nanoparticle(s), and the average concentration of the probes inside the gelatin nanoparticle(s) can be determined by XPS depth profile measurement. In XPS depth profile measurement, surface composition analysis can be performed sequentially while exposing the inside of the sample by using X-ray photoelectron spectroscopy (XPS) measurement and rare gas (such as argon) ion sputtering in combination. The distribution curve obtained by such measurement can be created, for example, with the vertical axis representing the atomic ratio (unit: at%) of each element and the horizontal axis representing the etching time (sputtering time). In the element distribution curve created in this manner whose horizontal axis is the etching time, the etching time substantially correlates with the distance from the surface. Accordingly, after elemental analysis from the surface of the gelatin nanoparticle(s) to the center thereof is performed to obtain the distribution curve of the elements of the gelatin nanoparticle(s), the amount of the probes in/on the surface layer can be obtained from the element distribution in a range from the measurement start point to the etching time corresponding to 0.01X (X is the average particle size), and the amount of the probes inside the gelatin nanoparticle(s) can be obtained from the element distribution in a range from the etching time corresponding to 0.01X to the etching time corresponding to the center thereof.

The amount of the probes is measured at a plurality of randomly selected points (for example, 10 points) by the above method, the average value (mass) of the probes contained in each of the surface layer and the inside is determined, and the concentration with respect to the total mass of gelatin particle(s) (that is, total mass of gelatin and probe) is obtained, thereby obtaining the average concentration of each of the surface layer and the inside. Gelatin nanoparticles are usually an aggregate composed of a plurality of particles, thus the average concentration of the probes can be a value obtained by summing and averaging the average concentrations of some gelatin particles (for example, 20 gelatin particles) selected from the above aggregate.

When the gelatin nanoparticles supporting the probes come into contact with cells, the gelatin nanoparticles are incorporated into the cells by the cells' own activities.

The cell may be any cell whose differentiation state is to be assessed, and preferably is a cell whose state is switched by differentiation or dedifferentiation between a state in which metabolism by glycolysis is predominant and a state in which metabolism in mitochondria is activated. Particularly preferred is a cell in which glycolysis is predominant in the undifferentiated state and metabolism in mitochondria is activated in the differentiated state. Examples of the cell include stem cells such as embryonic stem cells (ES cells) and induced pluripotent stem cells (iPS cells), immune cells such as monocytes and macrophages, nerve cells, and cancer cells.

For example, when cells or tissues derived from pluripotent stem cells induced to be differentiated are transplanted into a living body, tumor formation may occur when undifferentiated pluripotent stem cells remain. Therefore, introducing the above probes into pluripotent stem cells and assessing their differentiation state is expected to improve the safety of regenerative medicine such as transplantation.

In addition, in inflamed tissues, inflammatory macrophages M1 accumulate to induce inflammation, which is then subsided by anti-inflammatory macrophages M2. These macrophages are differentiated from monocytes, and it is said that change from macrophages M1 to macrophages M2 may occur. Metabolism by glycolysis is predominant in macrophages M1, and metabolism in mitochondria is more activated in macrophages M2. Therefore, introducing the above probes into monocytes and macrophages and assessing their differentiation state is expected to allow simplified determination of the state of the inflamed site and selection of an appropriate treatment at an early stage.

In place of undifferentiated cells, the cells may be differentiated somatic cells derived from biological samples or specimens extracted from various organs. Introducing the above probes into these cells and observing whether or not the expression of PDK1 is reduced allows assessing whether the cells become cancer cell or whether the cells obtain pluripotency due to dedifferentiation.

These cells are collected from a living body and the above probes are introduced into the cells by a known method. The above introduction may be performed by a known method such as an electroporation method or a microinjection method, but from the viewpoint of limiting a decrease in cell activity, a method in which the gelatin nanoparticles supporting the probes and the cells are mixed and cultured in a liquid is preferred.

### Acquisition of Signal from Probe (step S120)

In this next step, a signal derived from the above probes and emitted from the probe-introduced cells is acquired. This acquisition enables detection of the expression of PDK1 or Pdk1 in the cells.

The signal may be acquired by a method in accordance with the type of signal emitted from the probe. For example, when the probe contains a phosphor, the fluorescence emitted from the cell may be imaged by using a fluorescence microscope or the like to obtain a fluorescence image.

The acquisition of the signal may be performed by a method capable of confirming the presence or absence of the signal, or by a method that quantitatively measures the signal amount of the signal. The acquisition of the signal may be by a qualitative method or a quantitative method.

The signal may be acquired immediately after the probes are introduced, or may be acquired after a predetermined time has elapsed. In addition, the signal may be acquired only once, or may be acquired over time (continuously or multiple times at intervals). For determining the current state of the cells, the above signal may be acquired immediately after introducing the probes. For observing when the cells are differentiated, the signal may be acquired over time after introducing the probes.

In particular, introduction of the probes into the cells by using the gelatin nanoparticles supporting the probes facilitates the acquisition of the signal over time because gelatin nanoparticles slowly release the probes.

The cells are maintained in a viable state until the signal is acquired. During this state, the cells may be cultured in a medium or returned to the living body. The differentiation or dedifferentiation of the cells may be promoted or inhibited during this state.

### Assessment of Cell Differentiation State (step S130)

The differentiation state of a cell can be assessed based on the acquired signal.

According to the novel findings of the present inventors, the expression level of PDK1 changes in accordance with cell differentiation. When the cells are undifferentiated and metabolism by glycolysis is predominant, the expression level of Pdk1 or PDK1 is high. In contrast, when the cells are differentiated and metabolism in mitochondria is activated, the expression level of Pdk1 or PDK1 is low. Therefore, when the expression level of Pdk1 or PDK1 is higher, it can be determined that the cells are undifferentiated, and when the expression level of Pdk1 or PDK1 is lower, it can be determined that the cells are differentiated. Upon observing cells in which the probes are introduced over time, the cells can be determined to have differentiated when the expression level of Pdk1 or PDK1 decreases, and the cells can be determined to have dedifferentiated when the expression level of Pdk1 or PDK1 increases.

### Examples

Hereinafter, specific examples of the present invention will be described together with comparative examples, but the present invention is not limited thereto.

In the drawings related to the following description, "*" indicates that there is a significant difference (p value of less than 0.05 is regarded as statistically significant), and "ns" indicates that there is no significant difference.

The following experiments were performed by using a molecular beacon capable of detecting Pdk1 and, as a control, a molecular beacon capable of detecting mRNA of β-actin (Actb) that is constantly expressed regardless of the cell differentiation state.

### 1. Probe

The following probes were used.

Pdk1 MB: A probe in which the 5' end of sequence number 1 is modified with AlexaFlour488 and the 3' end with IBFQ (lowa black FQ)
Sequence number 1 is a molecular beacon in which sequences of positions 1 to 7 and positions 31 to 37 are complementary to each other and form the stem structure, and a sequence of positions 8 to 30 forms the loop structure.

Actb MB: A probe in which the 5' end of sequence number 2 is modified with TYE665 and the 3' end with IBRQ (lowa black RQ)
Sequence number 2 is a molecular beacon in which sequences of positions 1 to 6 and positions 24 to 30 are complementary to each other and form the stem structure, and a sequence of positions 7 to 23 forms the loop structure.

### 2. Probe

### 2-1. Preparation of Gelatin Nanoparticle

Gelatin (G-2613P, manufactured by Nitta Gelatin Inc.) was dissolved in 24 ml of a 0.1 M phosphate buffer aqueous solution (pH 5.0) at 37°C. To this solution, an appropriate amount of ethylenediamine was added. The pH of the solution was then adjusted to 5.0 by adding an aqueous hydrochloric acid solution. Further, an appropriate amount of 1-ethyl-3-(3-dimethylaminopropyl) carbodiimide hydrochloride was added, and the concentration of the gelatin was adjusted to 2 mass% by adding a 0.1 M phosphate buffer aqueous solution. This solution was stirred at 37°C for 4 hours to introduce ethylenediamine into the carboxyl group of the gelatin. The reaction mass was then dialyzed against redistilled water for 3 days to obtain a slurry of cationized gelatin. Subsequently, acetone as a phase separation inducer was added and mixed at 50°C to collect the particles precipitated in the slurry. The particles were washed with pure water to obtain cationized gelatin nanoparticles. These cationized gelatin nanoparticles are referred to as cGNS.

The apparent average particle size of cGNS, which was determined by the dynamic light scattering method at 37°C by using DLS-7000 manufactured by Otsuka Electronics Co., Ltd., was 168.0 nm. The zeta potential of cGNS, which was determined by the electrophoretic light scattering method by using DLS-8000 manufactured by Otsuka Electronics Co., Ltd., was 8.41 mV.

### 2-2. Supporting Molecular Beacon by Gelatin Nanoparticle

The cGNS and Pdk1 MB were mixed at room temperature for 15 minutes, then centrifuged and washed with water to obtain gelatin nanoparticles supporting the above probes. These gelatin nanoparticles are referred to as cGNS (Pdk1 MB).

The cGNS and Actb MB were mixed at room temperature for 15 minutes, then centrifuged and washed with water to obtain gelatin nanoparticles supporting the above probes. These gelatin nanoparticles are referred to as cGNS (Actb MB).

The amount of probes supported by each of cGNS (Pdk1 MB) and cGNS (Actb MB) was determined by a conventional method. The apparent average particle size and zeta potential of each of cGNS (Pdk1 MB) and cGNS (Actb MB) were determined in the same manner as for cGNS. Table 1 shows the results. The numbers shown in Table 1 indicate average ± standard deviation.

**Table 1**

| | Amount of supported probes (pmole/µg) | Average particle size (nm) | Zeta potential (mV) |
|---|---|---|---|
| cGNS (Pdk1 MB) | 19.7 ± 0.1 | 260.3 ± 13.0 | 7.46 ± 0.10 |
| cGNS (Actb MB) | 19.4 ± 0.2 | 252.8 ± 18.8 | 7.04 ± 0.35 |

As clearly shown in Table 1, no significant change was observed between the physical properties of cGNS (Pdk1 MB) and the physical properties of cGNS (Actb MB) depending on the type (sequence) of probe supported therein.

### 3. Test 1: ES cell

### 3-1. Comparison between Expression Level of Pdk1 and Expression Levels of Differentiation Marker Genes

Mouse ES cells (EB5, 2 × 10⁵ cells/well) were seeded in a 6-well plate and cultured for 48 hours in the presence of leukemia inhibitory factor (LIF) added thereto to maintain the undifferentiated state. Then, the medium was switched to OptiMEM, and the cells were further cultured under each of the condition such that LIF was added and the condition such that LIF was not added (i.e., the condition with LIF addition and the condition without LIF addition). At the time of culturing for 1, 2, and 3 days, cells were collected from the mediums, RNA was extracted, and cDNA was synthesized by reverse transcription. In addition, qRT-PCR was used to amplify Pdk1, the pluripotency markers Oct-3/4, Sox2 and Nanog, and the early differentiation markers, namely, Gata4, Gata6 and Sox17 (embryonic endoderm markers), T and GSC (embryonic mesoderm markers), Pax6 and Nestin (embryonic ectoderm markers), and Eomes and Cdx2 (embryonic trophectoderm markers). By the ΔΔCt method, Actb was first used as an internal standard to standardize the expression levels of mRNA of these markers, and further, the expression levels of the mRNA of these markers under the condition with LIF addition were standardized with respect to the expression levels of the mRNA of these markers under the condition without LIF addition.

FIG. 2A is a graph showing the expression levels of the mRNA of Pdk1 and undifferentiation markers, and FIG. 2B is a graph showing the expression levels of the mRNA of the early differentiation markers.

As clearly shown in FIGS. 2A and 2B, when cell differentiation was induced under the condition without the addition of LIF, the expression levels of the undifferentiation markers decreased significantly over time, and the expression levels of the early differentiation markers increased significantly. At the same time, the expression level of Pdk1 also significantly decreased over time.

These results show that the expression level of Pdk1 in ES cells changes in accordance with cell differentiation.

### 3-2. Observation of Changes in Pdk1 Expression Level depending on Cell Differentiation State

Mouse ES cells (EB5, 2 × 10⁵ cells/well) were seeded in a 6-well plate and cultured for 48 hours in the presence of leukemia inhibitory factor (LIF) added thereto to maintain the undifferentiated state. Then, the medium was switched to OptiMEM, and the cells were further cultured under each of the condition with LIF addition and the condition without LIF addition. At the time of culturing for 1, 2, and 3 days, 10 µg/mL of cGNS (Pdk1 MB) was added and co-cultured for 1 hour, and then the observation was performed under a fluorescence microscope.

After the medium was switched to OptiMEM, under the same conditions-with LIF addition and without LIF addition, 10 µg/mL of cGNS (Actb MB) was added at the time of culturing for 1, 2, and 3 days and co-cultured for 1 hour, and then the observation was performed under the fluorescence microscope.

FIG. 3A is a fluorescence image (right side) of the medium one day after the addition of cGNS (Pdk1 MB) under the condition with LIF addition and an image (left side) in which a bright field image and the fluorescence image are superimposed. FIG. 3B is a fluorescence image (right side) of the medium two days after the addition of cGNS (Pdk1 MB) under the condition with LIF addition and an image (left side) in which a bright field image and the fluorescence image are superimposed. FIG. 3C is a fluorescence image (right side) of the medium three days after the addition of cGNS (Pdk1 MB) under the condition with LIF addition and an image (left side) in which a bright field image and the fluorescence image are superimposed.

FIG. 4A is a fluorescence image (right side) of the medium one day after the addition of cGNS (Pdk1 MB) under the condition without LIF addition and an image (left side) in which a bright field image and the fluorescence image are superimposed. FIG. 4B is a fluorescence image (right side) of the medium two days after the addition of cGNS (Pdk1 MB) under the condition without LIF addition and an image (left side) in which a bright field image and the fluorescence image are superimposed. FIG. 4C is a fluorescence image (right side) of the medium three days after the addition of cGNS (Pdk1 MB) under the condition without LIF addition and an image (left side) in which a bright field image and the fluorescence image are superimposed.

FIG. 5A is a fluorescence image (right side) of the medium one day after the addition of cGNS (Actb MB) under the condition with LIF addition and an image (left side) in which a bright field image and the fluorescence image are superimposed. FIG. 5B is a fluorescence image (right side) of the medium two days after the addition of cGNS (Actb MB) under the condition with LIF addition and an image (left side) in which a bright field image and the fluorescence image are superimposed. FIG. 5C is a fluorescence image (right side) of the medium three days after the addition of cGNS (Actb MB) under the condition with LIF addition and an image (left side) in which a bright field image and the fluorescence image are superimposed.

FIG. 6A is a fluorescence image (right side) of the medium one day after the addition of cGNS (Actb MB) under the condition without LIF addition and an image (left side) in which a bright field image and the fluorescence image are superimposed. FIG. 6B is a fluorescence image (right side) of the medium two days after the addition of cGNS (Actb MB) under the condition without LIF addition and an image (left side) in which a bright field image and the fluorescence image are superimposed. FIG. 6C is a fluorescence image (right side) of the medium three days after the addition of cGNS (Actb MB) under the condition without LIF addition and an image (left side) in which a bright field image and the fluorescence image are superimposed.

As clearly shown in FIGS. 3A to 3C and 4A to 4C, when the cells were maintained in an undifferentiated state by adding LIF, fluorescence derived from Pdk1 MB continued to be expressed in almost all cells, but when cell differentiation was induced by not adding LIF, the number of cells whose fluorescence derived from Pdk1 MB was quenched increased over time.

On the other hand, as clearly shown in FIGS. 5A to 5C and 6A to 6C, no change was observed in the expression of fluorescence derived from Actb MB due to the change in the cell differentiation state that depends on whether LIF is added or not.

In the fluorescence image captured by the fluorescence microscope for each medium, the luminance of six randomly selected fields of view was measured, and the average of these luminance values was used as the fluorescence intensity of the fluorescence image.

FIG. 7A is a graph showing the fluorescence intensities of mediums with cGNS (Pdk1 MB) added thereto, and FIG. 7B is a graph showing the fluorescence intensities of mediums with cGNS (Actb MB) added thereto.

As clearly shown in FIG. 7A, regarding the fluorescence intensity when cGNS (Pdk1 MB) was introduced, the intensity from the cells whose differentiation was induced by not adding LIF increased over time as compared to the intensity from the cells whose undifferentiated state was maintained by adding LIF. In contrast, regarding the fluorescence intensity when cGNS (Actb MB) was introduced, there was no difference between the intensities from the cells whose differentiation was induced by not adding LIF and from the cells whose undifferentiated state was maintained by adding LIF, as clearly shown in FIG. 7B.

These results show that the expression level of Pdk1 in ES cells changes depending on the differentiation state of the cells. Therefore, the differentiation state of ES cells can also be determined by observing the expression level of Pdk1.

### 3-3. Evaluation of Difference in Signal Sensitivity depending on Probe Introduction Method

Mouse ES cells (EB5, 2 × 10⁵ cells/well) were seeded in a 6-well plate and cultured for 48 hours in the presence of leukemia inhibitory factor (LIF) added thereto to maintain the undifferentiated state. Then, the medium was switched to OptiMEM, and cGNS (Pdk1 MB) was added and co-cultured for 1 hour. In place of cGNS (Pdk1 MB), a complex of Lipofectamine 2000 (a gene transfer reagent composed of cationic lipid (liposomes)) and Pdk1 MB, or Pdk1 MB alone is added, and co-cultured for 1 hour in the same manner. The cells were then washed with PBS, further cultured for 6 hours, and the observation was performed under the fluorescence microscope.

In the same manner, after the medium was switched to OptiMEM, cGNS (Actb MB), a complex of Lipofectamine 2000 and Actb MB, or Actb MB alone was added and co-cultured for 1 hour. Subsequently, the cells were washed with PBS, and after further culturing for 6 hours, the cells were observed under the fluorescence microscope.

FIG. 8A is a fluorescence image (right side) of the medium with cGNS (Pdk1 MB) added thereto and an image (left side) in which a bright field image and the fluorescence image are superimposed. FIG. 8B is a fluorescence image (right side) of the medium with the complex of Lipofectamine 2000 and Pdk1 MB added thereto and an image (left side) in which a bright field image and the fluorescence image are superimposed. FIG. 8C is a fluorescence image (right side) of the medium with Pdk1 MB alone added thereto and an image (left side) in which a bright field image and the fluorescence image are superimposed.

FIG. 9A is a fluorescence image (right side) of the medium with cGNS (Actb MB) added thereto and an image (left side) in which a bright field image and the fluorescence image are superimposed. FIG. 9B is a fluorescence image (right side) of the medium with the complex of Lipofectamine 2000 and Actb MB added thereto and an image (left side) in which a bright field image and the fluorescence image are superimposed. FIG. 9C is a fluorescence image (right side) of the medium with Actb MB alone added thereto and an image (left side) in which a bright field image and the fluorescence image are superimposed.

As clearly shown in FIGS. 8A to 8C and 9A to 9C, when the gelatin nanoparticles supported the probes, a stronger signal (fluorescence) was observed than when the gene transfer reagent (Lipofectamine 2000) was used or when the probes were added alone.

When the gene transfer reagent (Lipofectamine 2000) was used, many dead cells were observed, but when the gelatin nanoparticles were used, almost no dead cells were observed.

These results show that when the gelatin nanoparticles support the probe, the number of probes that can be safely introduced into cells increases.

### 4. Test 2: Nerve cell

### 4-1. Comparison between Expression Level of Pdk1 and Expression Levels of Differentiation Marker Genes

Mouse ES cells (EB5, 2 × 10⁵ cells/well) were seeded in a 6-well plate and cultured for 48 hours in the presence of leukemia inhibitory factor (LIF) added thereto to maintain the undifferentiated state. Then, the medium was switched to a neural differentiation medium (NDiff227), and the cells were further cultured under each of the condition with LIF addition and the condition without LIF addition. At the time of culturing for 4, 7, and 9 days, cells were collected from the mediums, RNA was extracted, and cDNA was synthesized by reverse transcription. In addition, qRT-PCR was used to amplify pluripotency markers Oct-3/4, Sox2 and Nanog, neural progenitor cell markers Pax6 and Nestin, and a neuron marker Tubb III. By the ΔΔCt method, Actb was first used as an internal standard to standardize the expression levels of mRNA of these markers, and further, the expression levels of the mRNA of these markers under the condition with LIF addition were standardized with respect to the expression levels of the mRNA of these markers under the condition without LIF addition.

FIG. 10A is a graph showing the expression levels of the mRNA of Pdk1, FIG. 10B is a graph showing the expression levels of the mRNA of Oct-3/4, FIG. 10C is a graph showing the expression levels of the mRNA of Sox2, and FIG. 10D is a graph showing the expression levels of the mRNA of Nanog.

FIG. 11A is a graph showing the expression levels of the mRNA of Pax6, FIG. 11B is a graph showing the expression levels of the mRNA of Nestin, and FIG. 11C is a graph showing the expression levels of the mRNA of Tubb III.

As clearly shown in FIGS. 10A to 10D and 11A to 11C, when the differentiation into nerve cells was induced, the expression levels of the undifferentiation markers decreased significantly over time, and the expression levels of the neural progenitor differentiation markers and the neuron marker increased significantly. At the same time, the expression level of Pdk1 also significantly decreased over time.

These results show that the expression level of Pdk1 in nerve cells changes in accordance with the differentiation state of the cells.

### 4-2. Observation of Changes in Pdk1 Expression Level depending on Cell Differentiation State

Mouse ES cells (EB5, 2 × 10⁵ cells/well) were seeded in a 6-well plate and cultured for 48 hours in the presence of leukemia inhibitory factor (LIF) added thereto to maintain the undifferentiated state. Then, the medium was switched to a neural differentiation medium (NDiff227), and the cells were further cultured under each of the condition with LIF addition and the condition without LIF addition. At the time of culturing for 4, 7, and 9 days, 10 µg/mL of cGNS (Pdk1 MB) was added and co-cultured for 1 hour, and then the observation was performed under the fluorescence microscope.

After the medium was switched to a neural differentiation medium (NDiff227), under the same conditions-with LIF addition and without LIF addition, 10 µg/mL of cGNS (Actb MB) was added at the time of culturing for 4, 7 and 9 days, and co-cultured for 1 hour, and then the observation was performed under the fluorescence microscope.

FIG. 12A is a fluorescence image (right side) of the medium with cGNS (Pdk1 MB) added thereto under the condition with LIF addition and an image (left side) in which a bright field image and the fluorescence image are superimposed. FIG. 12B is a fluorescence image (right side) of the medium four days after the addition of cGNS (Pdk1 MB) under the condition without LIF addition and an image (left side) in which a bright field image and the fluorescence image are superimposed. FIG. 12C is a fluorescence image (right side) of the medium seven days after the addition of cGNS (Pdk1 MB) under the condition without LIF addition and an image (left side) in which a bright field image and the fluorescence image are superimposed. FIG. 12D is a fluorescence image (right side) of the medium nine days after the addition of cGNS (Pdk1 MB) under the condition without LIF addition and an image (left side) in which a bright field image and the fluorescence image are superimposed.

FIG. 13A is a fluorescence image (right side) of the medium with cGNS (Actb MB) added thereto under the condition with LIF addition and an image (left side) in which a bright field image and the fluorescence image are superimposed. FIG. 13B is a fluorescence image (right side) of the medium four days after the addition of cGNS (Actb MB) under the condition without LIF addition and an image (left side) in which a bright field image and the fluorescence image are superimposed. FIG. 13C is a fluorescence image (right side) of the medium seven days after the addition of cGNS (Actb MB) under the condition without LIF addition and an image (left side) in which a bright field image and the fluorescence image are superimposed. FIG. 13D is a fluorescence image (right side) of the medium nine days after the addition of cGNS (Actb MB) under the condition without LIF addition and an image (left side) in which a bright field image and the fluorescence image are superimposed.

As clearly shown in FIGS. 12A to 12D, when the cells were maintained in an undifferentiated state by adding LIF, fluorescence derived from Pdk1 MB was expressed in almost all cells, but when cell differentiation was induced by not adding LIF, the number of cells whose fluorescence derived from Pdk1 MB was quenched increased over time.

On the other hand, as clearly shown in FIGS. 13A to 13D, no change was observed in the expression of fluorescence derived from Actb MB due to the change in the cell differentiation state that depends on whether LIF is added or not.

In the fluorescence image captured by the fluorescence microscope for each medium, the luminance of six randomly selected fields of view was measured, and the average of these luminance values was used as the fluorescence intensity of the fluorescence image.

FIG. 14A is a graph showing the fluorescence intensities of mediums with cGNS (Pdk1 MB) added thereto, and FIG. 14B is a graph showing the fluorescence intensities of mediums with cGNS (Actb MB) added thereto. In FIGS. 14A and 14B, "Ctrl" represents the intensity from the medium in which an undifferentiated state was maintained by adding LIF, and "day 4," "day 7," and "day 9" represent the intensities from the mediums after 4, 7, and 9 days has passed after differentiation was induced by not adding LIF.

As clearly shown in FIG. 14A, regarding the fluorescence intensity when cGNS (Pdk1 MB) was introduced, the intensity from the cells whose differentiation was induced by not adding LIF increased over time as compared to the intensity from the cells whose undifferentiated state was maintained by adding LIF. In contrast, regarding the fluorescence intensity when cGNS (Actb MB) was introduced, there was no difference between the intensities from the cells whose differentiation was induced by not adding LIF and from the cells whose undifferentiated state was maintained by adding LIF, as clearly shown in FIG. 14B.

These results show that the expression level of Pdk1 in nerve cells changes depending on the differentiation state of the cells. Therefore, the differentiation state of nerve cells can also be determined by observing the expression level of Pdk1.

### 5. Test 3: Macrophage

### 5-1. Observation of Changes in Pdk1 Expression Level depending on Differentiation State of Standard Cell Line

Macrophage-like cell line (RAW264.7 cells, 2.0 × 10⁵ cells, M0) were seeded in 12-well plates with RPMI1640 medium and cultured. After 24 hours, 100 ng/ml lipopolysaccharide (LPS) and 20 ng/ml interferon y (IFN-y) were added to induce the cells to macrophages M1. Then, cGNS (Pdk1 MB) was added to the medium, and the observation was performed under the fluorescence microscope to find that fluorescence emission from all cells was observed.

Subsequently, 20 ng/ml of interleukin-4 (IL-4) and interleukin-13 were added to the medium, and the cells were cultured for 24 hours to induce the cells to macrophages M2. When the medium was observed under the fluorescence microscope, the fluorescence from the cells had disappeared.

These results show that the expression level of Pdk1 in a standard cell line of macrophages also changes depending on the differentiation state of the cells. Therefore, the differentiation state of immune cells such as macrophages can also be determined by observing the expression level of Pdk1.

### 5-2. Observation of Changes in Pdk1 Expression Level depending on Differentiation State of Mouse Peritoneal Macrophages

A 5 ml phosphate buffer aqueous solution was administered in the peritoneal cavity of Balb/c mouse, and the peritoneal cavity was thoroughly washed. The lavage fluid from the peritoneal cavity was collected, seeded in a 12-well plate, and cultured to obtain peritoneal macrophages. When the same experiment as the item 5-1 was performed by using the peritoneal macrophages, fluorescence emission from cells was observed when differentiation into macrophages M1 was induced, but when differentiation into macrophages M2 was induced, the fluorescence from the cells had disappeared.

These results show that the expression level of Pdk1 in macrophages derived from a living body also changes depending on the differentiation state of the cells. Therefore, the differentiation state of immune cells such as macrophages derived from a living body can also be determined by observing the expression level of Pdk1.

### 6. Test 4: Cancer cell

The same experiments as in Test 2 and Test 3 were performed on the colorectal cancer cell line. After the cells were induced to be differentiated to have a state in which metabolism by glycolysis was predominant, cGNS (Pdk1 MB) was added, and the observation was performed under the fluorescence microscope to find that fluorescence emission from all cells was observed. On the other hand, after a state, in which metabolism in mitochondria was activated, was established, the observation was performed under the fluorescence microscope to find that no fluorescence from cells was observed.

These test results show that the differentiation state of a wide variety of cell types can be determined by observing the expression level of Pdk1. These results also show that the differentiation state of a wide variety of cell types can be determined by observing the expression level of PDK1, and dedifferentiation of a wide variety of cell types can be determined by observing the expression level of Pdk1 or PDK1.

### Industrial Applicability

The present invention allows simplified observation of the differentiation state of cells. Therefore, the present invention can be applied to a wide variety of applications including regenerative medicine and disease detection and treatment, and is expected to contribute to the development of these fields.

## Claims

1. A method for assessing a differentiation state of a cell, the method comprising:
detecting mRNA of Pdk1 or pyruvate dehydrogenase kinase 1 (PDK1) in the cell, the mRNA encoding the pyruvate dehydrogenase kinase 1, wherein:
the detecting includes
introducing a probe capable of detecting the Pdk1 or the PDK1 into the cell, and
acquiring a signal from the introduced probe,
wherein the introducing of the probe includes bringing a gelatin nanoparticle that supports the probe,
which means that the probe is immobilised on the surface of or incorporated in the gelatin nanoparticle, into contact with the cell.

2. The method according to claim 1, wherein the detecting includes detecting the Pdk1 or the PDK1 over time.

3. The method according to claim 1 or 2, further comprising assessing the differentiation state of the cell based on a detection result of the Pdk1 or the PDK1.

4. The method according to claim 3, wherein the assessing includes determining whether the cell is in a state in which metabolism by glycolysis is predominant or in a state in which metabolism in mitochondria is activated.

5. The method according to any of claims 1 to 4, wherein the probe has a sequence complementary to at least a part of a nucleic acid sequence of the Pdk1.

6. The method according any of claims 1 to 5, wherein the probe is a molecular beacon.

7. The method according to any one of claims 1 to 6, wherein a state of the cell is switched by differentiation or dedifferentiation between a state in which metabolism by glycolysis is predominant and a state in which metabolism in mitochondria is activated.

8. The method according to any one of claims 1 to 7, wherein the cell is a stem cell.

9. The method according to any one of claims 1 to 7, wherein the cell is an immune cell.

10. The method according to any one of claims 1 to 7, wherein the cell is a cancer cell.

11. A gelatin nanoparticle for assessing a differentiation state of a cell, wherein:
the gelatin nanoparticle supports a probe capable of detecting mRNA of Pdk1 or pyruvate dehydrogenase kinase 1 (PDK1), the mRNA encoding the pyruvate dehydrogenase kinase 1.

12. The gelatin nanoparticle according to claim 11, wherein the probe has a sequence complementary to at least a part of a nucleic acid sequence of the Pdk1.

13. The gelatin nanoparticle according to claim 11 or 12, wherein the probe is a molecular beacon.

## Patentansprüche

1. Verfahren zur Beurteilung eines Differenzierungszustands einer Zelle, wobei das Verfahren umfasst:
Nachweisen von mRNA von Pdk1 oder Pyruvatdehydrogenasekinase 1 (PDK1) in der Zelle, wobei die mRNA für die Pyruvatdehydrogenasekinase 1 kodiert, wobei:
der Nachweis umfasst das Einbringen einer Sonde in die Zelle, wobei die Sonde in der Lage ist, Pdk1 oder PDK1 nachzuweisen, und
das Erfassen eines Signals von der eingeführten Sonde,
wobei das Einbringen der Sonde das Inkontaktbringen eines Gelatine-Nanopartikels in die Zelle umfasst, das die Sonde trägt, was bedeutet, dass die Sonde auf der Oberfläche des Gelatine-Nanopartikels immobilisiert oder in diesen eingebaut ist.

2. Verfahren nach Anspruch 1, wobei das Nachweisen von Pdk1 oder PDK1 über einen Zeitraum erfolgt.

3. Verfahren nach Anspruch 1 oder 2, das ferner das Bewerten des Differenzierungszustands der Zelle auf der Grundlage eines Nachweisresultats von Pdk1 oder PDK1 umfasst.

4. Verfahren nach Anspruch 3, wobei die Beurteilung das Bestimmen umfasst, ob sich die Zelle in einem Zustand befindet, in dem der Stoffwechsel durch Glykolyse vorherrscht, oder in einem Zustand, in dem der Stoffwechsel in den Mitochondrien aktiviert ist.

5. Verfahren nach einem der Ansprüche 1 bis 4, wobei die Sonde eine Sequenz aufweist, die zu mindestens einem Teil einer Nukleinsäuresequenz des Pdk1 komplementär ist.

6. Verfahren nach einem der Ansprüche 1 bis 5, wobei die Sonde ein molekularer Leuchtturm ist.

7. Verfahren nach einem der Ansprüche 1 bis 6, wobei ein Zustand der Zelle durch Differenzierung oder Entdifferenzierung zwischen einem Zustand, in dem der Stoffwechsel durch Glykolyse vorherrscht, und einem Zustand, in dem der Stoffwechsel in den Mitochondrien aktiviert ist, umgeschaltet wird.

8. Verfahren nach einem der Ansprüche 1 bis 7, wobei die Zelle eine Stammzelle ist.

9. Verfahren nach einem der Ansprüche 1 bis 7, wobei die Zelle eine Immunzelle ist.

10. Verfahren nach einem der Ansprüche 1 bis 7, wobei die Zelle eine Krebszelle ist.

11. Gelatine-Nanopartikel zur Beurteilung des Differenzierungszustands einer Zelle, wobei
das Gelatine-Nanopartikel eine Sonde trägt, die in der Lage ist, mRNA von Pdk1 oder Pyruvatdehydrogenase-Kinase 1 (PDK1) nachzuweisen, wobei die mRNA für die Pyruvatdehydrogenase-Kinase 1 kodiert.

12. Gelatine-Nanopartikel gemäß Anspruch 11, wobei die Sonde eine Sequenz aufweist, die zu mindestens einem Teil einer Nukleinsäuresequenz von Pdk1 komplementär ist.

13. Gelatine-Nanopartikel gemäß Anspruch 11 oder 12, wobei die Sonde ein molekularer Leuchtturm ist.

## Revendications

1. Procédé d'évaluation d'un état de différenciation d'une cellule, le procédé comprenant :
une détection de l'ARNm de Pdk1 ou de la pyruvate déshydrogénase kinase 1 (PDK1) dans la cellule, l'ARNm codant la pyruvate déshydrogénase kinase 1, dans lequel :
la détection inclut
une introduction d'une sonde capable de détecter le Pdk1 ou la PDK1 dans la cellule, et
une acquisition d'un signal provenant de la sonde introduite,
dans lequel l'introduction de la sonde inclut une mise en contact avec la cellule d'une nanoparticule de gélatine qui supporte la sonde, ce qui signifie que la sonde est immobilisée à la surface de ou incorporée dans la nanoparticule de gélatine.

2. Le procédé selon la revendication 1, dans lequel la détection inclut une détection de la Pdk1 ou de la PDK1 au fil du temps.

3. Le procédé selon la revendication 1 ou 2, comprenant en outre une évaluation de l'état de différenciation de la cellule sur la base d'un résultat de détection de la Pdk1 ou de la PDK1.

4. Le procédé selon la revendication 3, dans lequel l'évaluation inclut une détermination si la cellule est dans un état dans lequel un métabolisme par glycolyse est prédominant, ou dans un état dans lequel un métabolisme dans des mitochondries est activé.

5. Le procédé selon l'une quelconque des revendications 1 à 4, dans lequel la sonde a une séquence complémentaire d'au moins une partie d'une séquence d'acide nucléique de la Pdk1.

6. Le procédé selon l'une quelconque des revendications 1 à 5, dans lequel la sonde est une balise moléculaire.

7. Le procédé selon l'une quelconque des revendications 1 à 6, dans lequel un état de la cellule est modifié par différenciation ou dédifférenciation, entre un état dans lequel un métabolisme par glycolyse est prédominant, et un état dans lequel un métabolisme dans des mitochondries est activé.

8. Le procédé selon l'une quelconque des revendications 1 à 7, dans lequel la cellule est une cellule souche.

9. Le procédé selon l'une quelconque des revendications 1 à 7, dans lequel la cellule est une cellule immunitaire.

10. Le procédé selon l'une quelconque des revendications 1 à 7, dans lequel la cellule est une cellule cancéreuse.

11. Nanoparticule de gélatine pour évaluer un état de différenciation d'une cellule, dans laquelle :
la nanoparticule de gélatine supporte une sonde capable de détecter l'ARNm de Pdk1 ou de la pyruvate déshydrogénase kinase 1 (PDK1), l'ARNm codant la pyruvate déshydrogénase kinase 1.

12. La nanoparticule de gélatine selon la revendication 11, dans laquelle la sonde a une séquence complémentaire d'au moins une partie d'une séquence d'acide nucléique de la Pdk1.

13. La nanoparticule de gélatine selon la revendication 11 ou 12, dans laquelle la sonde est une balise moléculaire.
